# EUROPEAN PATENT SPECIFICATION

(11) **EP 1 988 820 B1**
(45) Date of publication and mention of the grant of the patent: **02.12.2015**
(21) Application number: 07705877.4
(22) Date of filing: 14.02.2007
(51) Int. Cl.: A61B 5/00, H04B 1/38, G04B 13/00, G06F 19/00, H04L 29/08

(54) **WIRELESS BODY SENSOR NETWORK**
DRAHTLOSES KÖRPERSENSORNETZWERK
RÉSEAU DE DÉTECTEURS CORPORELS SANS FIL

(30) Priority: 24.02.2006 EP 06110384
(43) Date of publication of application: 12.11.2008
(73) Proprietor: Koninklijke Philips N.V., 5656 AE Eindhoven (NL); Philips Intellectual Property & Standards GmbH, 20099 Hamburg (DE)
(72) Inventor: FALCK, Thomas, NL-5656 AA Eindhoven (NL)
(74) Representative: Verweij, Petronella Danielle
(86) International application number: PCT/IB2007/050483
(87) International publication number: WO 2007/096810

(56) References cited:
- EP-A- 1 220 501
- WO-A-2005/062232
- WO-A-2006/064397
- WO-A-2006/087670
- US-A1- 2005 288 738

## Description

This invention relates to communication devices especially devices adapted for use as body-worn medical sensors for continuously measuring ECG, SpO2, heart rate, blood pressure and the like, and in particular it relates to a method and apparatus for setting up wireless networks of such devices.

In order to continuously monitor patient body functions such as ECG, it is desirable to be able to mount sensors on the body of the patient, which are as light and compact as possible to avoid inconveniencing the patient, and are also capable of communicating wirelessly with each other. In addition, if a number of such sensors.are utilized on the same patient, some suitable means must be found for monitoring them all remotely in a coordinated fashion, and for this purpose it has been proposed to connect a plurality of sensors which are attached to the same patient, together in a wireless network. However, in practice, setting up wireless networks is difficult, using conventional technology, since the user has to configure network type, network name, network addresses, communication and security parameters for all the devices on the network. For example, on a network of personal computers running an operating system such as "Windows XP", the user must follow a series of detailed installation procedures, or create and propagate network settings using a "wizard".

Patent Application No. DE 020188 (Philips) discloses a Short-range Key transmission system for wireless consumer networks which provides a method of configuring network membership and bootstrapping the security system. German Patent Application No. DE 030119 discloses a device and method for controlling the addition/removal of wireless medical sensors to an existing wireless ad-hoc network and German Patent Application No. DE 030017 (Philips) discloses a method for automatically setting up an ad-hoc wireless network of Bluetooth enabled mobile devices.

All of these systems relate to wireless networks in which the signals are propagated externally of a patient, and this gives rise to a difficulty that systems employing wireless technologies designed to automate the establishment of networks, tend to make connections to all devices in the vicinity, whether or not they are in fact, connected to the same patient. For example, Bluetooth devices are frequently configured to automatically search for any new devices in range, and to establish connections with them.

EP 1220501 describes a method for the group creation for wireless terminals; WO 2005/ 062232 describes a patient network with wireless medical apparatuses and allocation thereof to a patient and his network; US 2005/0288738 describes a system and a method for RF wake-up of an implantable medical device; and WO 2006/087670 describes a device capable of being operated within a network using intra-body communication.

A communication device and a method according to the independent claims are provided.

Accordingly, the present invention seeks to provide a networking system for body mounted sensors which is able to restrict communication to sensors which are attached only to the same person. To this end, a wireless body area network ("BAN") according to an embodiment of the invention includes sensors or other electronic devices each of which is adapted to utilize body coupled communication ("BCC") to establish network connections, and also includes an RF transceiver for wireless short-range communication, once the network has been established.

It will be appreciated that the invention could be applied to any kind of networkable devices such as portable entertainment devices, which need to communicate data to each other or remote control devices for use with such devices.

In a typical arrangement, each device includes a BCC sensor which is capacitively coupled to the user, for example, in the manner described in US Patent No. 6211799, which describes a system for transmitting power and data through the user's body.

Various short-range communication technologies may be employed for the RF transceivers, such as Bluetooth, i.e. EE 802.15.4 (WPANs) and ZigBee. Preferably, each BCC sensor in the system is adapted to search the "wireless environment" as defined by the user's body, in order to discover any other sensors attached to the same person. In a preferred arrangement, once the BCC sensors have been "discovered", they are adapted to exchange network parameters so as to set up a wireless networks using their RF transceivers. Preferably, the system is so arranged that the individual RF transceivers can be put into a low powered sleep mode but can be woken, when required, by the BCC transceiver.

Accordingly, a preferred method of forming a wireless network, according to the invention, comprises the steps of:
a) Attaching a first wireless sensor to a patient;
b) Initiating a search by the sensor using BCC for any other sensors in the patient environment, for a predetermined period of time;
c) Initiating a sleep mode of the sensor if no other sensors are detected;
d) Attaching a second sensor to the patient;
e) Initiating a search, by the second sensor, for any other sensors by means of BCC and initializing an RF network set up procedure upon location of the first sensor;
f) Attaching a third sensor to the patient;
g) Repeating the search procedure using the new sensor, and thereby enabling the new sensor to join the existing RF network.

Preferably, when the second sensor is attached to the patient, and performs a search, it is arranged to transmit a "wake up signal", which triggers the first sensor to become active, to create the new wireless network, and to respond to the second sensor with information about the network parameters so as to enable the second sensor to use the parameters to join the network, and similarly, when any further sensor is attached to the patient, the reception of its search signal by the existing network causes one of the sensors already on the network to respond with the necessary network parameters.

As a result of this arrangement, the network is very easy to set up simply by attaching sensors to the body of a patient, and no manual network configuration is required. The network is established instantly, unlike (for example) a Bluetooth enquiry procedure which can take more than 10 seconds for each connection.

In a preferred arrangement of the invention, all sensors are identical and no separate dedicated network set up device is required, because each sensor device is capable of performing the required set up operations, depending upon results of its search for other devices. The system also has increased battery life time, compared to known systems because the power hungry radio circuit can remain in the sleep mode, until woken by the ultra-low powered BCC circuits. As a result of this, there is also reduced electro-magnetic pollution produced by the system.

The invention also extends to a sensor for use in monitoring a patient, comprising an RF transceiver for exchanging application data with other sensors, and also a BCC transceiver including appropriate controlling software for automatic network set up, and also to a wireless including a plurality of sensors according to the invention. In use, set up of the network is initiated through BCC, and once the network parameters have been exchanged, the radio sub-system can be woken up to establish a wireless ad-hoc network. Application data can then be transmitted through the wireless RF links between the devices.

One embodiment of the invention will now be described, with reference to the accompanying drawings, in which:
Fig. 1 is a schematic diagram of a network according to the invention;
Fig. 2 is a schematic diagram of an individual wireless body sensor; and
Fig. 3 is a detailed state chart of the communication sub-system of a wireless body sensor according to the invention.

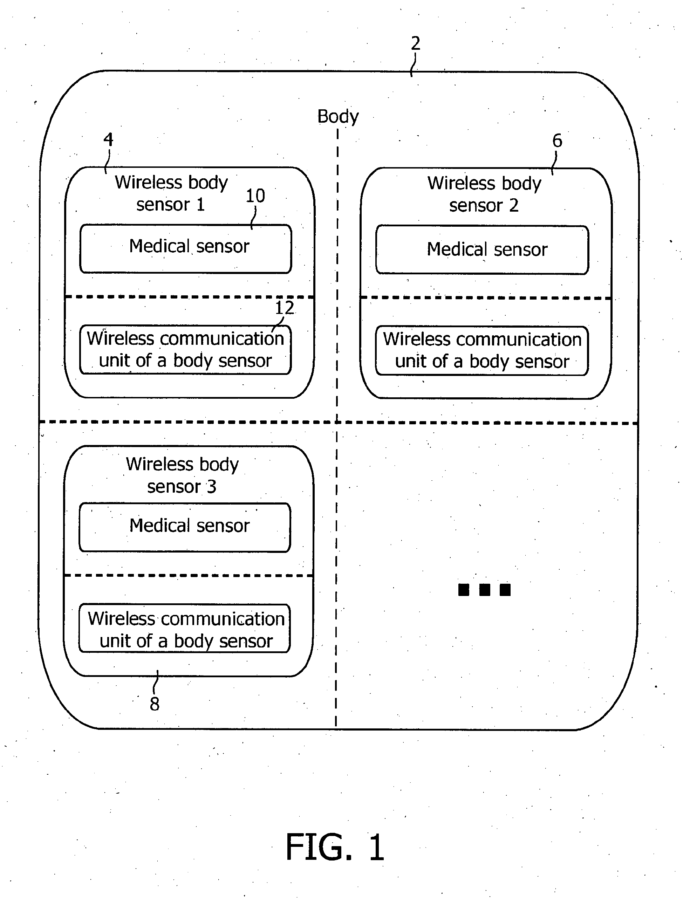

Referring to Figure 1, the outer rectangular area 2 represents the body of a patient, and each inner rectangular area 4, 6, 8, represents an individual wireless body sensor. As will be apparent from the drawing, each individual device consists of a medical sensor 10, and a wireless communication unit 12. The medical sensor may be of various kinds, such as an ECG or blood pressure measurement sensor.

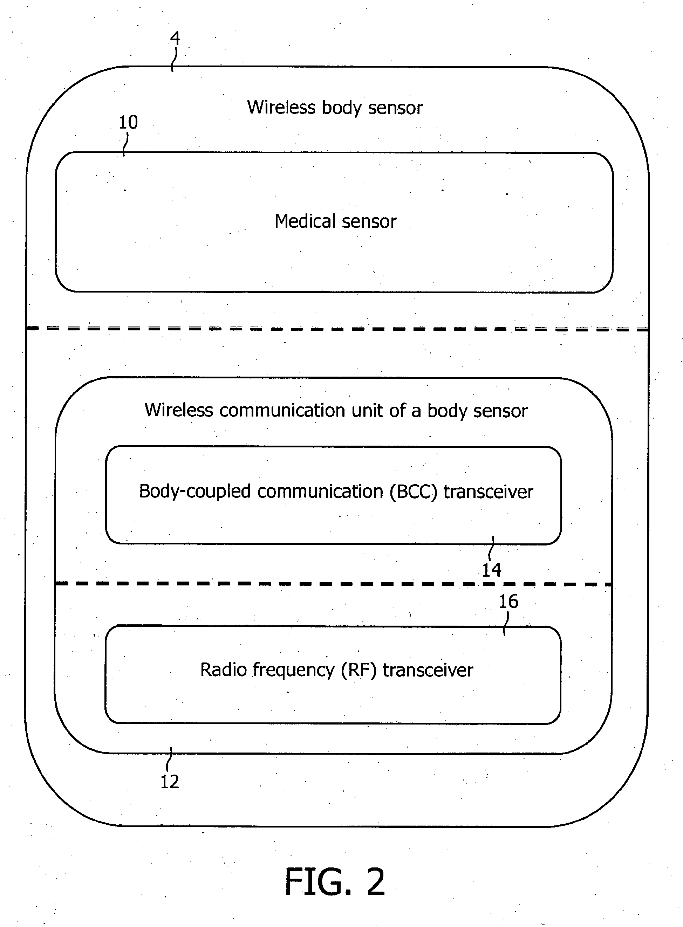

Referring to Figure 2, it will be seen that the wireless communication unit 12 consists of a body-coupled communication transceiver 14, which may consists of a "low frequency" transmitter operating between 100 KHz and 10MHz, of the kind described in US Patent No. 6211799. This is coupled capacitively to the body of the patient by means of suitable electrodes in contact with a portion of the patient's body.

The wireless communication unit also includes a conventional RF transceiver 16, for the short-range radio communication, such as a Bluetooth-type transceiver.

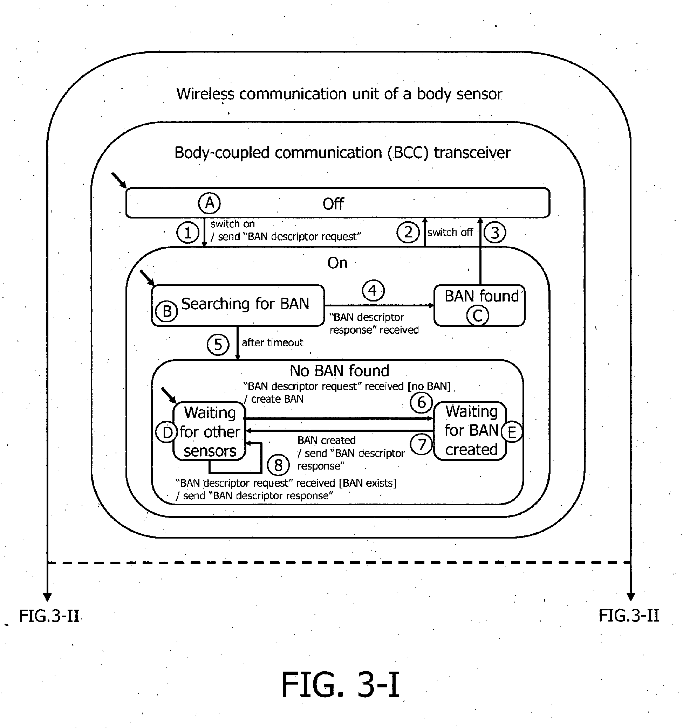

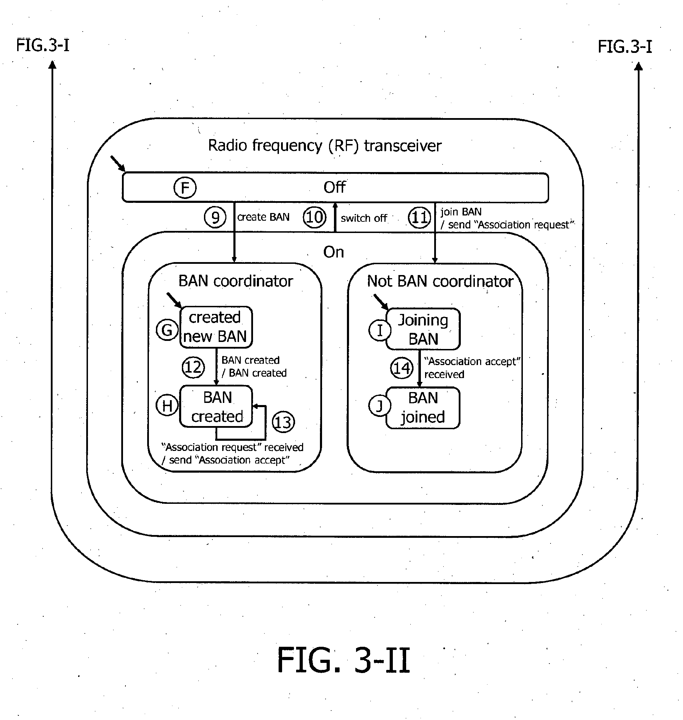

Figure 3 is a state chart showing the operation of the system in use, in which default states are marked with an arrow at their top left corner.

The stages of operation of the system are then as follows:
1. A first sensor is attached to a patient. Initially the BCC transceiver is in state A and the RF transceiver in state F.
2. The sensor is then switched on.

This triggers a transition 1 from state A to B: The sensor starts searching for other sensors on the body by sending a "BAN descriptor request" message through BCC.
3. Since it is the first sensor, there is no other sensor responding to the request. A timeout triggers transition 5 from state B to D, where the sensor waits for other sensors to be attached to the body.
4. A second sensor is attached to the patient. As before this sensor is in state A and F.
5. The second sensor is switched on. This triggers a transition 1 from state A to B: The sensor starts searching for other sensors on the body by sending a "BAN descriptor request" message through BCC.
6. Now the first sensor in state D receives the "BAN descriptor request" message and in turn signals its RF transceiver to create a body area network (BAN). It moves on to state E through transition 6, where it waits until the BAN has been created. (Note the condition "no BAN (=Body Area Network) exists"). The RF transceiver wakes up and goes from state F to G via transition 9. It creates a new BAN (this involves selecting a free channel and selecting a unique network identifier) and signals its BCC transceiver when the creation of the BAN has been completed. The RF transceiver thereby takes over the role of the coordinator of the BAN. Then the RF transceiver goes to state H, where it waits for incoming association requests of other sensors. The BCC transceiver moves from state E to D and sends through BCC to the second sensor the "BAN descriptor response" message containing the channel and network identifier of the newly created BAN. In state D the first sensor waits for further sensors being attached to the body.
7. The second sensor in state B receives the "BAN descriptor response" message containing the channel and network identifier of the network and moves to state C. The BCC transceiver instructs its RF transceiver to join the BAN specified by the channel and network identifier. The BCC transceiver goes to state A, i.e. is powered off. The RF transceiver of the second sensor wakes up and moves from state F to I. It sends an association request via RF to the first sensor to join the network specified by channel and network identifier.
8. The RF transceiver of the first sensor in state H receives the association request of the second sensor and sends through RF an "Association accept" message to the second sensor. The RF transceiver stays in state H, where it waits for further incoming association requests.
9. The RF transceiver of the second sensor in state I receives the "Association accept" message and moves to state J. Now the second sensor has successfully joined the BAN created by the first sensor.
10. A third sensor is attached to the patient. As before this sensor is in state A and F.
11. The third sensor is switched on. This triggers a transition 1 from state A to B: The sensor starts searching for other sensors on the body by sending a "BAN descriptor request" message through BCC.
12. Now the first sensor in state D receives the "BAN descriptor request" message and in turn responds via transition 8 with a "BAN descriptor response" message containing the channel and network identifier. (Note the condition "BAN (=Body Area Network) exists"). The sensor stays in state D and waits for further sensors being attached to the body.
13. The final steps for joining the network are the same as described in 7.-9. for the second sensor.
14. Attaching further sensors works in the same way as described for the third sensor in 10.-13.

Once monitoring data has been collected by the sensors on the BAN, it can be relayed to a bedside patient monitor (for example) which includes a similar RF communication module and a BCC arrangement. This is simply joined to the patients' own BAN by the patient touching the monitor, and thus activating the networking set up described above.

Alternatively the patient data can be transmitted by means of an additional patient-worn device including a BCC and a short-range radio similar to the other sensors, and also a wireless LAN or GPRS radio for external communication. In this way, the patient can be monitored within the hospital and/or outdoors.

## Claims

1. A communication device adapted for attachment to the body of a wearer so as to form part of a body area network ("BAN") comprising a body coupled communication ("BCC") device, and a short-range radio device, the device including means for detecting other similar communication devices on the wearer using BCC, and means for establishing a wireless network with other such devices using their short range radios, wherein the BCC device includes:
(a) means for discovering other similar devices attached to the same person;
(b) means for exchanging network parameters with similar devices; and
(c) means for sending a wake-up signal to a short-range radio device, which is configured to be in a low power sleep mode.

2. A communication device according to claim 1 in which the short-range radio device comprises a Bluetooth, Wifi or Zigbee device.

3. A medical sensor device incorporating a communication device according to any one of the preceding claims.

4. A network of communication devices including a plurality of communication devices according to any of claims 1 to 3.

5. A method of setting up a wireless body-area-network ("BAN") according to claim 4, comprising the steps of:
(a) using one BCC device to search for other devices;
(b) exchanging network parameters between BCC devices when they have contacted one another;
(c) utilizing the network parameters to set up a network between the radio devices.

6. A body area network ("BAN") of communication devices according to claim 4, and further comprising an external monitoring device which includes a BCC device and wireless communication means so that it can be connected to the BAN by contacting it to the body carrying the network.

## Patentansprüche

1. Kommunikationsgerät, das zum Anbringen an den Körper eines Trägers eingerichtet ist, um einen Teil eines körpernahem Netzwerks ("BAN") zu bilden, das ein körpergekoppeltes Kommunikations-("BCC-") Gerät sowie ein Kurzstreckenfunkgerät umfasst, wobei das Gerät Mittel zum Detektieren weiterer ähnlicher Kommunikationsgeräte an dem Träger unter Anwendung von BCC sowie Mittel zur Einrichtung eines drahtlosen Netzwerks mit anderen solchen Geräten unter Verwendung ihrer Kurzstreckenfunkgeräte enthält, wobei das BCC-Gerät umfasst:
(a) Mittel zum Auffinden weiterer ähnlicher an der gleichen Person angebrachter Geräte;
(b) Mittel zum Austausch von Netzwerkparametern mit ähnlichen Geräten; sowie
(c) Mittel zur Übertragung eines Wecksignals zu einem Kurzstreckenfunkgerät, das so eingerichtet ist, dass es sich in einem Sleep-Mode mit geringem Stromverbrauch befindet.

2. Kommunikationsgerät nach Anspruch 1, bei dem das Kurzstreckenfunkgerät aus einem Bluetooth-, Wifi- oder Zigbee-Gerät besteht.

3. Medizinischer Sensor, in den ein Kommunikationsgerät nach einem der vorangegangenen Ansprüche integriert ist.

4. Netzwerk mit mehreren Kommunikationsgeräten nach einem der Ansprüche 1 bis 3.

5. Verfahren zum Aufbau eines körpernahen Netzwerks ("BAN") nach Anspruch 4, das die folgenden Schritte umfasst, wonach:
(a) ein BCC-Gerät zum Suchen nach weiteren Geräten verwendet wird;
(b) Netzwerkparameter zwischen BCC-Geräten ausgetauscht werden, wenn diese miteinander in Kontakt stehen;
(c) die Netzwerkparameter zum Aufbau eines Netzwerks zwischen den Funkgeräten verwendet werden.

6. Körpernahes Netzwerk ("BAN") von Kommunikationsgeräten nach Anspruch 4, weiterhin umfassend ein externes Überwachungsgerät, das ein BCC-Gerät sowie drahtlose Kommunikationsmittel enthält, so dass es mit dem BAN verbunden werden kann, in dem ein Kontakt mit dem das Netzwerk tragenden Körper hergestellt wird.

## Revendications

1. Dispositif de communication adapté pour fixation au corps d'une personne afin de faire partie d'un réseau corporel (« Body Area Network ou « BAN ») comprenant un dispositif de communication à couplage corporel (« Body Coupled Communication » ou « BCC »), et un dispositif radio à faible portée, le dispositif incluant des moyens pour détecter d'autres dispositifs de communication similaires sur la personne utilisant le BCC, et des moyens pour établir un réseau sans fil avec d'autres tels dispositifs en utilisant leurs radios à faible portée, dans lequel le dispositif BCC inclut :
(a) des moyens pour découvrir d'autres dispositifs similaires fixés à la même personne ;
(b) des moyens pour échanger des paramètres de réseau avec des dispositifs similaires ; et
(c) des moyens pour envoyer un signal de réveil à un dispositif radio à faible portée, qui est configuré pour être dans un mode de sommeil à basse puissance.

2. Dispositif de communication selon la revendication 1, dans lequel le dispositif radio à faible portée comprend un dispositif Bluetooth, Wifi ou Zigbee.

3. Dispositif capteur médical incorporant un dispositif de communication selon l'une quelconque des revendications précédentes.

4. Réseau de dispositifs de communication incluant une pluralité de dispositifs de communication selon l'une quelconque des revendications 1 à 3.

5. Procédé d'établissement d'un réseau corporel (« BAN ») sans fil selon la revendication 4, comprenant les étapes de :
(a) l'utilisation d'un dispositif BCC pour rechercher d'autre dispositifs ;
(b) l'échange de paramètres de réseau entre des dispositifs BCC lorsqu'ils se sont contactés ;
(c) l'utilisation des paramètres de réseau pour établir un réseau entre les dispositifs radio.

6. Réseau corporel (« BAN ») de dispositifs de communication selon la revendication 4, et comprenant en outre un dispositif de surveillance externe qui inclut un dispositif BCC, et des moyens de communication sans fil pour qu'il puisse être connecté au BAN en le mettant en contact avec le corps portant le réseau.
